(19) Europäisches Patentamt
European Patent Office
Office européen des brevets

(11) EP 3 411 364 B1

(12) # EUROPÄISCHE PATENTSCHRIFT

(45) Veröffentlichungstag und Bekanntmachung des Hinweises auf die Patenterteilung:
**22.01.2020 Patentblatt 2020/04**

(21) Anmeldenummer: **17701882.7**

(22) Anmeldetag: **30.01.2017**

(51) Int Cl.:
***C07D 317/24*** *(2006.01)* ***C07D 319/06*** *(2006.01)*

(86) Internationale Anmeldenummer:
**PCT/EP2017/051906**

(87) Internationale Veröffentlichungsnummer:
**WO 2017/134002 (10.08.2017 Gazette 2017/32)**

(54) **VERFAHREN ZUR HERSTELLUNG VON MONOMEREN AUS ISOMERENMISCHUNGEN**
METHOD FOR PRODUCING MONOMERS FROM ISOMERIC MIXTURES
PROCÉDÉ DE PRODUCTION DE MONOMÈRES À PARTIR DE MÉLANGES D'ISOMÈRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.02.2016 DE 102016201660**

(43) Veröffentlichungstag der Anmeldung:
**12.12.2018 Patentblatt 2018/50**

(73) Patentinhaber: **Evonik Operations GmbH**
**45128 Essen (DE)**

(72) Erfinder:
- **BEYER, Silvia**
  **64372 Ober-Ramstadt (DE)**
- **DAVE, Gaurang**
  **Mumbai 400055 (IN)**
- **DILLMANN, Hans-Jürgen**
  **64560 Riedstadt (DE)**
- **HERZOG, Volker**
  **64297 Darmstadt (DE)**
- **KNEBEL, Joachim**
  **64665 Alsbach-Hähnlein (DE)**
- **MERBACH, Ralf**
  **64572 Buettelborn (DE)**
- **SCHÜTZ, Thorben**
  **64665 Alsbach-Hähnlein (DE)**
- **TROCHA, Martin**
  **45136 Essen (DE)**
- **KÖMMELT, Sabine**
  **64285 Darmstadt (DE)**
- **HARTMANN, Patrik**
  **64572 Buettelborn (DE)**

(74) Vertreter: **Evonik Patent Association**
**c/o Evonik Industries AG**
**IP Management**
**PB 84/339**
**Rodenbacher Chaussee 4**
**63457 Hanau (DE)**

(56) Entgegenhaltungen:
**GB-A- 690 709**
**JP-A- 2004 018 389**
**JP-A- 2014 044 415**
**US-A- 4 975 519**

- **N. GARCA, ET AL.: "Comparative study of the relaxation behaviour of acrylic polymers with flexible cyclic groups in their structure", POLYMER, Bd. 41, Nr. 17, August 2000 (2000-08), Seiten 6603-6611, XP004196880, Elsevier Science Publishers, Amsterdam, NL ISSN: 0032-3861, DOI: 10.1016/S0032-3861(99)00889-7**

EP 3 411 364 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gebiet der Erfindung

Die Erfindung betrifft ein Verfahren zur Herstellung von (Meth)acrylsäureestern von isomeren Alkoholen, erhalten durch Reaktion von Glycerin mit Formaldehyd (hydroxiisubstituierte Dioxane und Dioxolane), in hoher Ausbeute unter Verwendung von Katalysatoren auf Titan,- Zirkon,- oder Zinnbasis, oder basenkatalysiert.

Stand der Technik

Die Herstellung von (Meth)acrylsäureestern von hydroxymodifizerten Dioxolanen durch Umesterung ist bekannt.

In der JP 2004018389 wird ein Verfahren zur Herstellung von (Meth)acrylsäureestern beschrieben. Als Ausgangsstoff wird ketalisiertes Glycerol, ein 5-Ring-Derivat, mit Alkyl(meth)acrylat in Anwesenheit von Titan- oder Zinnkatalysatoren umgesetzt. Anschließend wird das erhaltene (Meth)acrylat mit Wasser in Anwesenheit eines Kationenaustauscherharzes weiter umgesetzt.

In der JP 2004059435 wird ein ähnliches Verfahren zur Darstellung von Dioxolanverbindungen beschrieben. Es werden nur primäre Alkohole (5-Ring) beschrieben.

Die Glasübergangstemperaturen der Homopolymeren dieser 5-Ring-Derivate liegen bei ca. 40°C.

Für eine Anwendung z.B. in Bodenbeschichtungen nach DE102011109139 (Evonik) ist jedoch eine hohe Glastemperatur des Polymeren von Vorteil, da dadurch eine hohe mechanische Belastbarkeit gewährleistet wird. Die Literatur zeigt, dass hier das von der Sechsringstruktur abgeleitete Monomer 5- Methacryloyloxy-1,3- dioxan von Vorteil ist, da es eine Glastemperatur von ca. 115 °C bewirkt (Guzman, J. et al., Macromolecular Symposia 2003, 191, 177-190; Guzman, J. J. Polymer Sci. Part B 2002, 40(11), 1154-1162)).

GB 690709 beschreibt ein Verfahren zur Herstellung von Estern der (Meth)acrylsäure und 4-Methyloldioxolanen und deren Derivaten. Es wird eine Veresterung beschrieben, bei der Methacrylsäure mit isomeren Alkoholen, erhalten durch Reaktion von Glycerin mit Formaldehyd (hydroxysubstituierte Dioxane und Dioxolane), umgesetzt wird. Insbesondere wird 4-(Methacryloyloxymethyl)-1,3-dioxolan aus dem Glycerinformal-Isomerengemisch durch Ver- oder Umesterung mit Methacrylsäure, Methacrylsäurechlorid oder Methylmethacrylat erhalten.

Dabei entsteht nur das Fünfringmethacrylat, obwohl der Rohstoff aus dem Gemisch der Fünf- und Sechsringisomeren besteht (ca. 25:75 %). Der Sechsringrohstoff erweist sich als unreaktiv, bleibt unumgesetzt und wird bei der Aufreinigung des Monomeren abgetrennt.

In Beispiel 1 wird dazu ausgeführt, dass das im Isomerengemisch der Ausgangstoffe enthaltene 1,3-Methylidenglycerol nicht umgesetzt wird und daher am Ende der Reaktion destillativ entfernt werden muss.

Das gleiche Verhalten findet man bei der Herstellung des 4-(Acryloyloxymethyl)-1,3-dioxolans aus Acrylsäure und Glycerinformal-Isomerengemisch durch Veresterung (H. Minato u.a., Bull. Chem. Soc. Jpn., 42 (4), 1146 (1969)).

US 4975519 und JP 2014044415 beschreiben ein Verfahren zur Herstellung von einem Gemisch von 4-(Methacryloyloxymethyl)-1,3-dioxolan und 5-Methacryloyloxy-1,3-dioxan durch Umsetzung von Glycerinformal-Isomerengemisch mit Methacrylsäureanhydrid.

Ausgehend vom großtechnisch verfügbaren Isomerengemisch wird der Fachmann davon weggeführt, eine Umsetzung zu den entsprechenden Estern durchzuführen, ohne vorher das weniger reaktive 6-Ring-Isomer abzutrennen.

Aufgabe und Lösung

Aufgabe war es, ein Verfahren zu entwickeln, welches die zuvor dargestellten Nachteile überwindet.

Glycerinformal, ein Isomerengemisch aus 5-Ring-Verbindungen und 6-Ring-Verbindungen, ist CMR-gelistet. Glycerinformalmethacrylat ist nicht CMR-gelistet. Aufgabe war es, das Edukt Glycerinformal möglichst vollständig umzusetzen.

Aufgabe war es, ein Monomerengemisch mit niedrigem Dampfdruck herzustellen im Vergleich zu Methylmethacrylat (Dampfdruck 3,7 kPa bei 20°C, Yaws' Handbook of Antoine Coeff. For Vapour Pressure, Electronic ISBN: 978-1-59124-879-8) oder Styrol (0, 6 kPa bei 20°C, Yaws' Handbook of Antoine Coeff. For Vapour Pressure, Electronic ISBN: 978-1-59124-879-8).

Aufgabe war es, ein Homopolymer zur Verfügung zu stellen, welches vergleichbare Glasübergangstemperaturen aufweist, wie sie mit dem üblicherweise verwendeten Methylmethacrylat und Styrol erreicht werden, und somit einen Austausch des geruchsintensiven Methylmethacrylats oder gesundheitsgefährdenden Styrols zu ermöglichen.

Überraschenderweise erhält man aus dem kommerziell erhältlichen Glycerinformal ein (Meth)acrylatestergemisch mit hohem Sechsringanteil, wenn man den Rohstoff in Gegenwart eines Titanalkoholats, z.B. Isopropyltitanat, eines Zirkonkomplexes, z.B. Zirkonacetylacetonat, einer Zinnverbindung wie Dioctylzinnoxid oder eines Gemischs aus

Lithiumhydroxid und Calciumoxid mit Alkyl(meth)acrylat umsetzt.

Glycerinformal beschreibt im Rahmen dieser Erfindung das Isomerengemisch von Alkoholen, erhalten durch die Reaktion von Glycerin mit Formaldehyd. Das hier verwendete handelsübliche Isomerengemisch liegt bei einem Verhältnis 5-Ring zu 6-Ring von ca. 20% zu 80% bis 70% zu 30%.

Es wurde gefunden, dass der Sechsring im (Meth)acrylatestergemisch vorliegt. Dies garantiert eine ausreichend hohe Glastemperatur des Polymeren. Zudem erhält man eine Monomermischung mit einem sehr geringen Restalkoholgehalt.

Es wurde gefunden, dass bei dem erfindungsgemäß erhaltenen (Meth)acrylat vergleichbare Tg von mind. 75°C erzielt werden, die einen Austausch gegen derzeit verwendete Methacrylate und Styrol ermöglichen.

Überraschend wurde gefunden, dass der Dampfdruck des erfindungsgemäß hergestellten Glycerinformal-Gemisches bei Raumtemperatur in der Größenordnung 0,01 hPa liegt.

Überraschend wurde gefunden, dass die erfindungsgemäßen Monomermischungen ideale Ausgangsstoffe für Polymerisate sind, die Anwendung in Reaktionsharzen sowie daraus hergestellten Kaltplastiken finden. Sie zeichnen sich durch sehr gute Eigenschaften aus, wie Langlebigkeit, mechanischen Eigenschaften, Abriebfestigkeit, u.U. Weißgrad oder Pigmentierung und Griffigkeit, die sämtlich mindestens so gut wie bei Systemen des Standes der Technik sind. Darüber hinaus zeichnen sich diese Systeme jedoch gegenüber dem Stand der Technik durch eine besonders geringe Geruchsbildung bzw. Freisetzung volatiler Bestandteile aus.

Die erfindungsgemäßen Monomermischungen eignen sich hervorragend für Polymere, die in Beschichtungen, beispielsweise Bodenbeschichtungen, Farben und Lacke, Straßenmarkierungen, Gießharze, Bautenschutz, Composit-Werkstoffen, Beschichtungs- oder Laminierungsmitteln, beispielsweise für Orthopädieharze, aber auch in Klebstoffen eingesetzt werden.

Überraschenderweise wurde gefunden, dass durch eine Umesterungsreaktion von Alkyl(meth)acrylaten mit isomeren Alkoholen, erhalten durch Reaktion von Glycerin mit Formaldehyd (hydroxisubstituierte Dioxane und Dioxolane), besonders vorteilhaft in Gegenwart von Katalysatoren auf Titanbasis (Meth)acrylsäureester sowohl der hydroxisubstituierten Dioxane als auch der hydroxisubstituierten Dioxolane gleichzeitig in hoher Ausbeute dargestellt werden können.

Im erfindungsgemäßen Verfahren werden Alkyl(meth)acrylate mit Glycerinformal, in Gegenwart von 0,01 bis 10 Gew.-%, insbesondere von 0,1 bis 5 Gew.-% bezogen auf das gesamte Reaktionsgemisch, eines Katalysators auf Titanbasis, umgesetzt.

Die Edukte und Katalysatoren

Als Alkyl(meth)acrylat können beispielsweise eingesetzt werden: Ethyl(meth)acrylat, n-Propyl(meth)acrylat, i-Propyl(meth)acrylat, n-Butyl(meth)acrylat, i-Butyl(meth)acrylat, tert.-Butyl(meth)acrylat, n-Pentyl(meth)acrylat, n-Hexyl(meth)acrylat, oder bevorzugt Methylmethacrylat sowie Butylacrylat.

Die Schreibweise (Meth)acrylat bedeutet hier sowohl Methacrylat, wie z.B. Methylmethacrylat, Ethylmethacrylat usw., als auch Acrylat, wie z.B. Methylacrylat, Ethylacrylat usw., sowie Mischungen aus beiden.

Die für die Umesterung eingesetzten Alkohole sind isomere Alkohole erhalten durch Reaktion von Glycerin mit Formaldehyd (hydroxisubstituierte Dioxane und Dioxolane), wobei das Verhältnis von Dioxanen zu Dioxolanen von 20:80 bis 70:30 mol% reicht.

Als Katalysatoren sind Zinn-, Zirkon- oder Titanverbindungen oder anorganische Basen geeignet. Es können Umesterungskatalysatoren auf Titanbasis, besonders Tetramethoxy titan, Tetraethoxy titan, Tetra-iso-propoxy titan, Tetra-n- butoxytitan, Tetrakis (2-ethylhexyl oxy) titan, Tetrastearyl oxytitan eingesetzt werden.

Bevorzugt sind Katalysators aus der Gruppe Titanalkoholate, insbesondere Isopropyltitanat, Zirkonkomplexe, insbesondere Zirkonacetylacetonat, Zinnverbindungen, insbesondere Dioctylzinnoxid, oder eines Gemisches aus Lithiumhydroxid und Calciumoxid.

Die Umsetzung von Alkyl(meth)acrylaten mit isomeren Alkoholen wird bei Temperaturen zwischen 30 und 180 °C, vorzugsweise zwischen 50 und 130 °C durchgeführt. Die Umsetzung erfolgt in Gegenwart von 0,01 bis 10 Gew.-%, bevorzugt von 0,1 bis 5 Gew.-%, besonders bevorzugt von 0,2 bis 2 Gew.-%, eines Katalysators, bezogen auf die gesamte Reaktionsmischung.

Formal reagieren äquimolare Mengen an Alkyl(meth)acrylat mit den isomeren Alkoholen zu den gewünschten Endprodukten. In der Praxis ist es jedoch zweckmäßig, während der Umsetzung die Alkyl(meth)acrylate im Überschuß zu halten, wobei die Alkyl(meth)acrylate in Mengen von 1,2 bis 15 Mol, vorzugsweise 2 bis 10 Mol, pro Mol Hydroxylgruppen eingesetzt werden.

Zur Vermeidung von durch Polymerisation der Alkyl(meth)acrylate verursachten Ausbeuteverlusten ist es zweckmäßig, die Umsetzung und Aufarbeitung des Reaktionsgemisches in Gegenwart von Polymerisationsinhibitoren durchzuführen.

Die Polymerisationsinhibitoren

Polymerisationsinhibitoren sind bereits bekannt. So können beispielsweise 1,4-Dihydroxybenzole zur Stabilisierung zugegeben werden. Es können jedoch auch anders substituierte Dihydroxybenzole zum Einsatz kommen. Allgemein lassen sich derartige Inhibitoren mit der allgemeinen Formel (I) wiedergeben

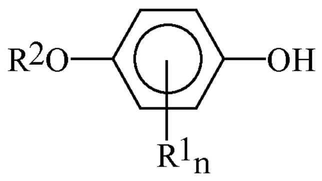

(I)

worin

$R^1$ Wasserstoff, einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert-Butyl, Cl, F oder Br;

n eine ganze Zahl im Bereich von eins bis vier, vorzugsweise eins oder zwei ist; und

$R^2$ Wasserstoff, einen linearen oder verzweigten Alkylrest mit eins bis acht Kohlenstoffatomen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl oder tert-Butyl.

Es können jedoch auch Verbindungen mit 1,4-Benzochinon als Stammverbindung eingesetzt werden. Diese lassen sich mit der Formel (II) beschreiben

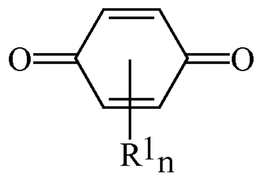

(II)

worin

$R^1$ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert-Butyl, Cl, F oder Br; und

n eine ganze Zahl im Bereich von eins bis vier, vorzugsweise eins oder zwei ist.

Ebenso werden Phenole der allgemeinen Struktur (III) eingesetzt.

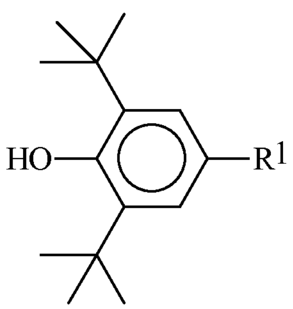

(III)

worin
$R^1$ einen linearen oder verzweigten Alkylrest mit eins bis acht Kohlenstoffatomen, Aryl oder Aralkyl, Proprionsäureester

mit 1 bis 4 wertigen Alkoholen, welche auch Heteroatome wie S, O und N enthalten können, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert-Butyl, bedeutet.

Eine weitere vorteilhafte Substanzklasse stellen sterisch gehinderte Phenole auf Basis von Triazinderivaten der Formel (IV) dar:

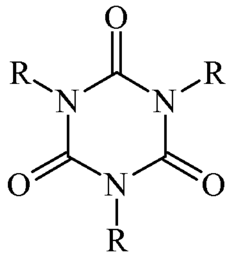

(IV)

mit R = Verbindung der Formel (V)

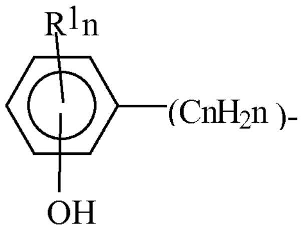

(V)

worin

$R^1 = C_nH_{2n+1}$
mit n = 1 oder 2 ist.

Eine weitere Gruppe von bekannten Inhibitoren sind Amine, insbesondere sterisch gehinderte Amine.

Zu diesen gehören insbesondere Phenylendiamine, die durch Formel (VI) darstellbar sind

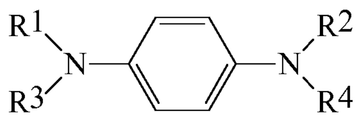

(VI),

worin $R^1$, $R^2$, $R^3$ und $R^4$ unabhängig Wasserstoff sowie Alkyl-, Aryl-, Alkaryl-, Aralkyl-Reste mit jeweils bis zu 40, vorzugsweise bis zu 20 Kohlenstoffatomen darstellen, wobei vorzugsweise mindestens einer der Reste $R^1$, $R^2$, $R^3$ und $R^4$ Wasserstoff ist.

Beispielhafte p-Phenylendiamine umfassen p-Phenylendiamin worin die Reste $R^1$, $R^2$, $R^3$ und $R^4$ Wasserstoff sind; N-Phenyl-N'-alkyl-p-phenylendiamine wie beispielsweise, N-Phenyl-N'-methyl-p-phenylendiamin, N-Phenyl-N'-ethyl-p-phenylendiamin, N-Phenyl-N'-propyl-p-phenylendiamin, N-Phenyl-N'-isopropyl-p-phenylendiamin, N-Phenyl-N'-n-butyl-p-phenylendiamine, N-Phenyl-N'-isobutyl-p-phenylendiamin, N-Phenyl-N'-sec-butyl-p-phenylendiamin, N-Phenyl-N'-tert-butyl-p-phenylendiamin, N-Phenyl-N'-n-pentyl-p-phenylendiamin, N-Phenyl-N'-n-hexyl-p-phenylendiamin, N-Phenyl-N'-(1-methylhexyl)-p-phenylendiamin, N-Phenyl-N'-(1,3-dimethylbutyl)-p-phenylendiamin, N-Phenyl-N'-(1,4-dimethyl-pentyl)-p-phenylendiamin; N-Phenyl-N',N'-dialkyl-p-phenylendiamine, wie beispielsweise N-Phenyl-N',N'-dimethyl-p-phenylendiamin, N-Phenyl-N',N'-diethyl-p-phenylendiamin, N-Phenyl-N',N'-di-n-butyl-p-phenylendiamin N-Phenyl-N',N'-di-sec-butyl-p-phenylendiamin, N-Phenyl-N'-methyl-N'-ethyl-p-phenylendiamin; N,N-Dialkyl-p-phenylendiamine, wie beispielsweise N,N-Dimethyl-p-phenylendiamin und N,N'-Diethyl-p-phenylendiamin; N,N'-Dialkyl-p-phenylendiamine, wie beispielsweise N,N'-Diisopropyl-p-phenylendiamin, N,N'-Diisobutyl-p-phenylendiamin; N,N'-Diaryl-phenylendiamine, wie beispielsweise N,N'-Diphenyl-p-phenylendiamin; N,N,N'-Trialkyl-p-phenylendiamine, wie beispielsweise N,N,N'-Trimethyl-p-phenylendiamin, N,N,N'-Triethyl-p-phenylendiamin.

Darüber hinaus bilden Phenazin-Farbstoffe eine weitere bevorzugte Gruppe. Diese umfassen insbesondere Indulin und Nigrosin. Nigrosin entsteht durch Erhitzen von Nitrobenzol, Anilin und salzsaurem Anilin mit metallischem

Eisen und $FeCl_3$. Hierbei sind alkohollösliche Anilinfarbstoffe bevorzugt, die beispielsweise 5 Benzolkerne umfassen können, wie Dianilido-N,N-diphenylphenosafranin. Diese Stoffe sind weithin bekannt und können kommerziell erhalten werden.

Geeignet sind auch Dialkylhydroxylamine wie z.B. N,N-Diethylhydroxylamin.

Besonders erfolgreich werden die Verbindungen 1,4-Dihydroxybenzol, 4-Methoxyphenol, 2,5-Dichloro-3,6-dihydroxy-1,4-benzochinon, 1,3,5-Trimethyl-2,4,6-tris-(3,5-di-tert.butyl-4-hydroxybenzyl)benzol, 2,6-Di-tert. butyl-4-methylphenol, 2,4-Dimethyl-6-tert. butylphenol, 2,2-Bis [3,5-Bis(1,1-dimethylethyl)-4-hydroxyphenyl-1-oxopropoxymethyl)] 1,3-propandiylester, 2,2'-Thiodiethylbis-[3-(3,5-di-tert.butyl-4-hydroxyphenyl)]propionat, Octadecyl-3-(3,5-di-tert.butyl-4-hydroxyphenyl)propionat, 3,5-Bis(1,1-dimethylethyl-2,2-Methylenbis-(4-methyl-6-tert.butyl)phenol, Tris-(4-tert.butyl-3-hydroxy-2,6-dimethylbenzyl)-s-triazin-2,4,6-(1H,3H,5H)trion, Tris (3,5-ditert.butyl-4-hydroxy)-s-triazin-2,4,6-(1H,3H,5H) trion, tert.-Butyl-3,5-dihydroxybenzol oder Diphenyl-p-phenylendiamin (DPPD) sowie N-oxyl-Derivate wie z.B. 4-Hydroxy-2,2,6,6-tetramethylpiperidin-1-oxyl, 2,2,5,5-Tetramethyl-3-oxopyrrolidin-1-oxyl oder 2,2,6,6-Tetra eingesetzt, wobei hiervon wiederum ganz besonders zweckmäßig Hydrochinonmonomethylether (4-Methoxyphenol) ist.

Besonders bevorzugte Inhibitoren sind Phenothiazin, Hydrochinonmonomethylether und 4-Hydroxy-2,2,6,6-tetramethylpiperidyl-N-oxyl.

Die genannten Inhibitoren sind kommerziell erhältlich.

Als Grundstabilisierung für ethylenisch ungesättigte Verbindungen können die genannten Verbindungen allein oder in Mischung von zwei oder mehr Verbindungen eingesetzt werden. Sofern es sich um phenolische Verbindungen handelt, ist die Anwesenheit von Sauerstoff im Reaktionsgemisch erforderlich, um eine ausreichende Wirksamkeit gegen Polymerisation sicherzustellen. Dabei ist die Verwendung von Luft als Sauerstoffquelle besonders bevorzugt.

Lösungsmittel

Weiterhin ist es möglich, die Umsetzung in Gegenwart eines inerten Lösungsmittels oder von Lösungsmittelgemischen durchzuführen.

Als Lösungsmittel können alle inerten organischen Lösungsmittel verwendet werden, wie beispielsweise aliphatische Kohlenwasserstoffe, wie Hexan, Heptan, Oktan, Cyclohexan oder Gemische von aliphatischen Kohlenwasserstoffen, wie beispielsweise Petrolether, Ligroin, Dekalin, oder Benzin.

Ferner können aromatische Lösungsmittel, wie beispielsweise Benzol, Toluol oder die isomeren Xylole und Gemische aus vorstehend genannten Verbindungen, eingesetzt werden.

Ferner kommen sauerstoffhaltige Kohlenwasserstoffe in Frage, wie beispielsweise Diethylenglycolmonomethylether, Diethylenglycolmonoethylether, Triethylenglycolmonomethylether, Triethylenglycolmonoethylether, Diethylenglycolmonobutylether, Diethylether oder Methyl-tertbutylether. Auch Wasser kommt als inertes Lösungsmittel in Betracht. Besonders bevorzugt werden beispielsweise Toluol oder Cyclohexan als Lösungsmittel verwendet.

Die Umsetzung kann unter Normaldruck, Unter- oder Überdruck durchgeführt werden, wobei die Reaktionsführung diskontinuierlich oder kontinuierlich erfolgen kann. Bevorzugt wird bei Normaldruck umgesetzt.

Vorteilhafte Reaktionsbedingungen zur Abtrennung des Azeotrops sind Drücke zwischen 1 und 500 hPa.

Im Allgemeinen werden die Edukte, Alkyl(meth)acrylat und Glycerinformal gemeinsam in Gegenwart eines Inhibitors oder Inhibitorgemisches auf Reaktionstemperatur erhitzt und entwässert. Nach dem Entwässerungsschritt wird der Katalysator bzw. das Katalysatorgemisch sowie Alkyl(meth)acrylat zugegeben.

In Abhängigkeit von der Qualität der Ausgangsstoffe, kann auf den Entwässerungsschritt verzichtet werden. Ein geringer Wassergehalt in den Edukten kann bei der Umsetzung toleriert werden. Kontinuierlich werden der abgespaltene Alkohol sowie der überschüssige Alkyl(meth)acrylat, vorzugsweise gemeinsam als Azeotrop, abdestilliert. Die Reaktionszeiten liegen im Allgemeinen zwischen 1 und 20 Stunden, vorzugsweise zwischen 2 und 12 Stunden, und hängen von der Reaktionstemperatur bzw. von der eingesetzten Menge des Katalysators ab.

Nach Beendigung der Umsetzung wird das überschüssige Alkyl(meth)acrylat teilweise oder vorzugsweise vollständig, beispielsweise durch Abdestillieren, aus dem Reaktionsprodukt entfernt.

Anschließend wird über eine weitere Destillation das Produkt vom Katalysator getrennt und weiter aufgereinigt. Gegebenenfalls kann alternativ ein Filtrationsschritt vor der Destillation zur Abtrennung des Katalysators eingefügt werden. Je nach Anforderungen an die Reinheit des Reaktionsproduktes kann auch auf die Destillation verzichtet werden, nachdem der Katalysator abfiltriert wurde.

Das erfindungsgemäße Verfahren zur Herstellung von (Meth)acrylsäureestern durch Umesterung von Alkyl(meth)acrylaten mit isomeren Alkoholen, erhalten durch Reaktion von Glycerin mit Formaldehyd (hydroxisubstituierte Dioxane und Dioxolane), führt zu deutlich höhere Ausbeuten als die Verfahren des Standes der Technik.

Mit dem erfindungsgemäßen Verfahren werden Monomergemische erhalten, aus denen Homopolymere mit einer Glasübergangstemperatur Tg>75°C erhalten werden können. Die Glasübergangstemperaturen liegen vorzugsweise im Bereich zwischen 75 und 90°C.

Mit dem erfindungsgemäßen Verfahren werden Monomergemische erhalten, deren Dampfdruck bei Raumtemperatur

< 5 hPa beträgt. Vorzugsweise liegt der Dampfdruck bei Raumtemperatur bei 0,001 bis 5 hPa, besonders bevorzugt bei 0,005 bis 0,05 hPa.

Die folgenden Beispiele sollen die Erfindung erläutern.

BEISPIELE

Beispiel 1: Zirkonkatalysierte Umesterung

Die Reaktionsmischung aus 375 g Glycerinformal, 1009 g Methylmethacrylat (MMA), 0,12 g Hydrochinonmonomethylether, 0,16 g Phenothiazin, wird in einem 2 l-Kolben mit 50 cm-Glaskolonne und Kolonnenpackungen der Fa. Sulzer, Rührung und Lufteinleitrohr eingewogen und unter Lufteinleitung zum Sieden erhitzt. Eventuell vorhandenes Wasser wird azeotrop mit 49g Destillat aus dem Ansatz entfernt. Nach Abkühlen werden 11,3g $Zr(acac)_4$ und 49 g MMA zugegeben. Der Ansatz wird erneut zum Sieden erhitzt.

Das entstehende Methanol/MMA-Gemisch wird bei 100-120°C Sumpftemperatur kontinuierlich ausgeschleust. Während der Reaktion wird MMA über einen Zulauf dem Destillat massenäquivalent zudosiert (insgesamt 237 g). Nach 9:20 h ist die Reaktion beendet.

Überschüssiges MMA und andere Leichtsieder werden bei 120°C Sumpftemperatur und 8 mbar abdestilliert. Man erhält 645 g Rohester mit einem Gehalt der isomeren Methacrylsäureester von 97,2% und einem Restgehalt der Glycerinformalisomeren von ca. 0,3%. Nach abschließender fraktionierender Destillation werden 593g Produkt erhalten. Der Anteil des Isomerengemisches von 4-(Methacryloyloxymethyl)-1,3-dioxolan und 5- Methacryloyloxy-1,3- dioxan liegt bei 99,8%. Der Anteil Isomerengemisch Glycerinformal liegt bei 0,14%.

Beispiel 2: Titanatkatalysierte Umsetzung

Die Reaktionsmischung aus 29,1 kg Glycerinformal, 78,5 kg Methylmethacrylat (MMA), 9,6 g Hydrochinonmonomethylether, 1,4g 4-Hydroxy-2,2,6,6-tetramethylpiperidin-1-oxyl, 2,8g N,N-Diethylhydroxylamin (85% w/w in Wasser), 12 g Phenothiazin, wird in einem 145 l-Emaillekessel mit 4,5 m-Glaskolonne NW150 und Kolonnenpackungen der Fa. Sulzer, Rührung und Lufteinleitrohr ohne Katalysator eingewogen und unter Lufteinleitung mittels Ölumlaufthermostat zum Sieden erhitzt. Der Ansatz wird bei einem niedrigen Rücklaufverhältnis ohne Temperaturbegrenzung mit 1700 g Destillat entwässert. Nach Erreichen einer konstanten Kolonnenkopftemperatur von 99 °C wird der Entwässerungsschritt beendet.

Dem Ansatz werden 729 g Isopropyltitanat und 1700 g MMA hinzugefügt. Es wird das entstehende Methanol/MMA-Gemisch bei 100 -120°C Sumpftemperatur kontinuierlich ausgeschleust. Während der Reaktion wird MMA über einen Zulauf dem Destillat massenäquivalent zudosiert (insgesamt 12,6 kg). Nach 8:30 h steigt die Kolonnenkopftemperatur langsam an und nach 9:30 Stunden sind 99 °C im Kolonnenkopf erreicht. Wenn die Temperatur im Kolonnenkopf 99 °C übersteigt und dann konstant bleibt, ist die Reaktion beendet.

Nun wird zum Abtrennen des überschüssigen MMA der Ansatz bei 350 mbar Druck zum Sieden gebracht und Methylmethacrylat abdestilliert, während der Druck kontinuierlich weiter gesenkt wird, um eine Sumpftemperatur von 75-85°C zu halten. Wenn ein Druck von 32 bis 38 mbar erreicht ist, wird der Ansatz noch 45 Min. bei 85°C gehalten. Insgesamt werden 58,35 kg Methylmethacrylat erhalten. Der im Sumpf verbleibende katalysatorhaltige Rohester umfaßt 48,2 kg und besteht aus 94,5 % Isomerengemisch von 4-(Methacryloyloxymethyl)-1,3-dioxolan und 5- Methacryloyloxy-1,3-dioxan sowie 2,8% MMA und 1,22% Glycerinformal-Isomerengemisch. Er kann durch Destillation bei 0,4-0,5 mbar und einer Ölumlauftemperatur von 165-170°C an einem Dünnschichtverdampfer DS 50 der Fa. NGW vom Katalysator befreit und so weiter gereinigt werden. Das Destillat besteht aus den isomeren Methacrylsäureestern und besitzt eine gaschromatographisch ermittelte Reinheit von 98,6%. Der Anteil der Glycerinformalisomeren beträgt 0,68%.

Die Glasübergangstemperatur des Isomerengemisches liegt bei 75°C. Dieser Wert zeigt, dass vom Isomerengemisch sowohl der 5-Ring, als auch der 6-Ring umgesetzt wurden.

Beispiel 3: Zinnkatalysierte Umesterung

Die Reaktionsmischung aus 375 g Glycerinformal, 1009 g Methylmethacrylat (MMA), 0,12 g Hydrochinonmonomethylether, 0,16 g Phenothiazin, wird in einem 2 l-Kolben mit 50 cm-Glaskolonne und Kolonnenpackungen der Fa. Sulzer, Rührung und Lufteinleitrohr eingewogen und unter Lufteinleitung zum Sieden erhitzt. Eventuell vorhandenes Waser wird azeotrop mit 60 g Destillat aus dem Ansatz entfernt. Nach Abkühlen werden 18,8 g Dioctylzinnoxid und 60 g MMA zugegeben. Der Ansatz wird erneut zum Sieden erhitzt.

Das entstehende Methanol/MMA-Gemisch bei 100-120°C Sumpftemperatur kontinuierlich ausgeschleust. Während der Reaktion wird MMA über einen Zulauf dem Destillat massenäquivalent zudosiert (insgesamt 275g). Nach 11:00 h ist die Reaktion beendet.

Überschüssiges MMA und andere Leichtsieder werden bei 120°C Sumpftemperatur und 8 mbar abdestilliert. Man erhält 550 g Rohester mit einem Gehalt der isomeren Methacrylsäureester von 95,6% und einem Restgehalt der Glycerinformalisomeren von ca. 0,3%. Nach abschließender fraktionierender Destillation werden 490 g Produkt erhalten. Der Anteil des Isomerengemisches von 4-(Methacryloyloxymethyl)-1,3-dioxolan und 5- Methacryloyloxy-1,3- dioxan beträgt 98,25%. Der Anteil Isomerengemisch Glycerinformal beträgt 0,17%.

Beispiel 4: Basenkatalysierte Umesterung

Die Reaktionsmischung aus 5,6 kg Glycerinformal, 12,5 Methylmethacrylat (MMA), 1,9 g Hydrochinonmonomethylether sowie 19,7 g CaO und 8,4g LiOH als Katalysator, wird in einem 20 l-Kessel mit 1 m-Glaskolonne NW50 und Kolonnenpackungen der Fa. Sulzer, Rührung und Lufteinleitrohr eingewogen und unter Lufteinleitung zum Sieden erhitzt.

Das entstehende Methanol/MMA-Gemisch bei 100-120°C Sumpftemperatur kontinuierlich ausgeschleust. Während der Reaktion wird MMA über einen Zulauf dem Destillat massenäquivalent zudosiert (insgesamt 3,5 kg). Nach ca. 5 h Reaktionszeit werden 9,8g CaO und 4,2g LiOH und 200g MMA zugegeben. Nach 11:30 h ist die Reaktion beendet.

Überschüssiges MMA und andere Leichtsieder werden zunächst am Kessel und dann am Dünnschichtverdampfer (5-6 mbar, 110°C Ölumlauftemperatur 110°C) abdestilliert. Man erhält nach abschließender Druckfiltration 8kg eines Gemisches aus den isomeren Methacrylsäureestern und mit einer gaschromatographisch ermittelten Reinheit von 94,7%. Der Anteil der Glycerinformalisomeren beträgt 0,96%.

Beispiel 5: Durchführung Plattenpolymerisation

Es wurden 200g Glycerinformalmethacrylat mit 0,1% Azobis(isobutyronitril) (AIBN) initiiert und in eine Glaskammer (25x25cm; 0,4cm Schrumpfschnur) gefüllt.Die Kammer wurde luftdicht verschlossen und im Wasserbad auspolymerisiert (12h 50°C; 3h 60°C) und im Anschluss bei 90°C im Trockenschrank für 2 Stunden getempert. Das hergestellte Homopolymer Polyglycerinformalmethacrylat wurde anschließend zur Ermittlung der Glasübergangstemperatur genutzt.

Beispiel 6: DSC-Messung (Bestimmung der Glasübergangstemperatur)

Zur Bestimmung der Glasübergangstemperatur wird als ein gängiges Messverfahren die dynamische Differenzkalorimetrie (Differential Scanning Calorimetry, DSC) verwendet (Ehrenstein, Gottfried W. Riedel, Gabriela, Trawiel, Pia (2004); Thermal Analysis of Plastics-Theory and Practice; Hanser Publishers).

Die mittels Plattenpolymerisation (siehe Beispiel 5) hergestellte Probe wurde im Temperaturbereich von -50 bis 150°C mit einer Heizrate von 10K/min (Schnelles Kühlen) im Aluminiumtiegel mit perforiertem Deckel unter Stickstoff vermessen (DSC-Messgerät von Mettler Toledo mit FlüssigStickstoff-Kühlung).

Ermittelte Glasübergangstemperatur Polyglycerinformalmethacrylat: $T_g$= 85°C

Beispiel 7: Bestimmung der Polymerisationszeit (PZ-Messung)

Für die Bestimmung der Polymerisationszeit wurden 0,2 Gew-% AIBN (CAS 78-67-1) eingewogen und im Monomer Glycerinformalmethacrylat gelöst. Diese Mischung wurde für 2 min mit einem Magnetrührer homogenisiert, in ein Reagenzglas (18 x 180 mm) umgefüllt, auf T=60°C im Wasserbad erhitzt und anschließend die Polymerisationszeit gemessen. Mit Hilfe eines Temperaturfühlers wurde der Temperaturverlauf der Reaktion aufgezeichnet. Dieser Temperaturfühler befindet sich in einem zweiten, kleineren und mit Diethylenglycol als Trägerflüssigkeit gefüllten Röhrchen, das in der Mitte des Reagenzglases so fixiert ist, dass es tief genug in die Probenflüssigkeit eintaucht, um eine genaue Messung der Probentemperatur zu ermöglichen. Als Start der Messung gilt der Zeitpunkt bei der die gewählte Reaktionstemperatur (T=60°C) erreicht wird. Die Position der maximalen Reaktionstemperatur $T_{max}$ entspricht der Polymerisationszeit.

PZ-Messung Glycerinformalmethacrylat

| | | |
|---|---|---|
| Initiator: 0,2 % AIBN | Zeit [min] | 102,5 |
| T=60°C | $T_{max}$ [°C] | 114,5 |

Beispiel 8: Bestimmung des Dampfdrucks (dynamische Methode mittels Ebulliometer)

Die Dampfdruckdaten wurde mit der dynamischen Methode mittels Ebulliometer (Eigenentwicklung der LTP GmbH für kleine Probenmengen ab ca. 20ml in Anlehnung an die Richtlinie der EU: Dynamic Method of the EC A.4 Vapor Pressure guideline auf Basis der OECD Richtlinie 104) ermittelt.

Experimentelle Daten: Dampfdruck Glycerinformalmethacrylat

| T [°C] | Druck [hPa] |
|---|---|
| 56,45 | 0,53 |
| 64,16 | 1 |
| 71,33 | 1,8 |
| 80,89 | 3,4 |
| 90,35 | 6,5 |
| 96,96 | 9,4 |
| 108,95 | 17,5 |
| 122,31 | 33,9 |
| 134,72 | 58,4 |
| 144,76 | 88,9 |
| 195,88 | 479,3 |
| 201,10 | 551,3 |
| 208,21 | 654,0 |
| 215,35 | 778,8 |
| 224,18 | 697,3 |
| 226,17 | 1004,1 |

Antoine-Parameter (DDB-Format) für Glycerinformalmethacrylat

$$\log (P/\text{Torr}) = A - B / (C + T/°C)$$

| A | B | C |
|---|---|---|
| 6,39262 | 1232,98 | 125,136 |

Dampfdruck bei T=20°C für Glycerinformalmethacrylat (extrapoliert aus Antoine-Parameter): p= 0,01 hPa

## Patentansprüche

1. Verfahren zur Herstellung von (Meth)acrylatestergemischen, **dadurch gekennzeichnet, dass** ein Glycerinformal-Isomerengemisch in Gegenwart eines Katalysators mit Alkyl(meth)acrylat umgesetzt wird.

2. Verfahren zur Herstellung von (Meth)acrylatestergemischen gemäß Anspruch 1, **dadurch gekennzeichnet, dass** ein Glycerinformal-Isomerengemisch mit einem Isomerenverhältnis von 5-Ring- zu 6-Ring-Isomer von 20:80 bis 70:30 eingesetzt wird.

3. Verfahren gemäß Anspruch 2, **dadurch gekennzeichnet, dass** Homopolymere aus dem (Meth)acrylatestergemisch

eine Glasübergangstemperatur Tg zwischen 75°C und 90°C aufweisen.

4. Verfahren zur Herstellung von (Meth)acrylatestergemischen gemäß Anspruch 1, **dadurch gekennzeichnet, dass** in Gegenwart eines Katalysators aus der Gruppe Titanalkoholate, Zirkonkomplexe, Zinnverbindungen oder eines Gemisches aus Lithiumhydroxid und Calciumoxid umgesetzt wird.

5. Verfahren zur Herstellung von (Meth)acrylatestergemischen gemäß Anspruch 1, **dadurch gekennzeichnet, dass** bei einer Temperatur zwischen 30 und 180°C umgesetzt wird.

## Claims

1. Process for producing (meth)acrylate ester mixtures, **characterized in that** a glycerol formal isomer mixture is reacted with alkyl (meth)acrylate in the presence of a catalyst.

2. Process for producing (meth)acrylate ester mixtures according to Claim 1, **characterized in that** a glycerol formal isomer mixture having an isomer ratio of 5-membered ring isomer to 6-membered ring isomer of 20:80 to 70:30 is employed.

3. Process according to Claim 2, **characterized in that** homopolymers of the (meth)acrylate ester mixture have a glass transition temperature Tg between 75°C and 90°C.

4. Process for producing (meth)acrylate ester mixtures according to Claim 1, **characterized in that** the reaction is performed in the presence of a catalyst from the group titanium alkoxides, zirconium complexes, tin compounds or a mixture of lithium hydroxide and calcium oxide.

5. Process for producing (meth)acrylate ester mixtures according to Claim 1, **characterized in that** the reaction is performed at a temperature between 30°C and 180°C.

## Revendications

1. Procédé pour la préparation de mélanges d'ester de (méth)acrylate, **caractérisé en ce qu'**un mélange d'isomères de glycérine-formal est transformé en présence d'un catalyseur avec du (méth)acrylate d'alkyle.

2. Procédé pour la préparation de mélanges d'ester de (méth)acrylate selon la revendication 1, **caractérisé en ce qu'**un mélange d'isomères de glycérine-formal comportant un rapport d'isomères d'isomère à cycle à 5 chaînons à isomère à cycle à 6 chaînons de 20 :80 à 70 :30, est utilisé.

3. Procédé selon la revendication 2, **caractérisé en ce que** des homopolymères du mélange d'ester de (méth)acrylate présentent une température de transition vitreuse $T_v$ comprise entre 75 °C et 90 °C.

4. Procédé pour la préparation de mélanges d'ester de (méth)acrylate selon la revendication 1, **caractérisé en ce que** la transformation a lieu en présence d'un catalyseur du groupe des alcoolates de titane, des complexes de zirconium, des composés de l'étain, ou d'un mélange d'hydroxyde de lithium et d'oxyde de calcium.

5. Procédé pour la préparation de mélanges d'ester de (méth)acrylate selon la revendication 1, **caractérisé en ce que** la transformation a lieu à une température comprise entre 30 et 180 °C.

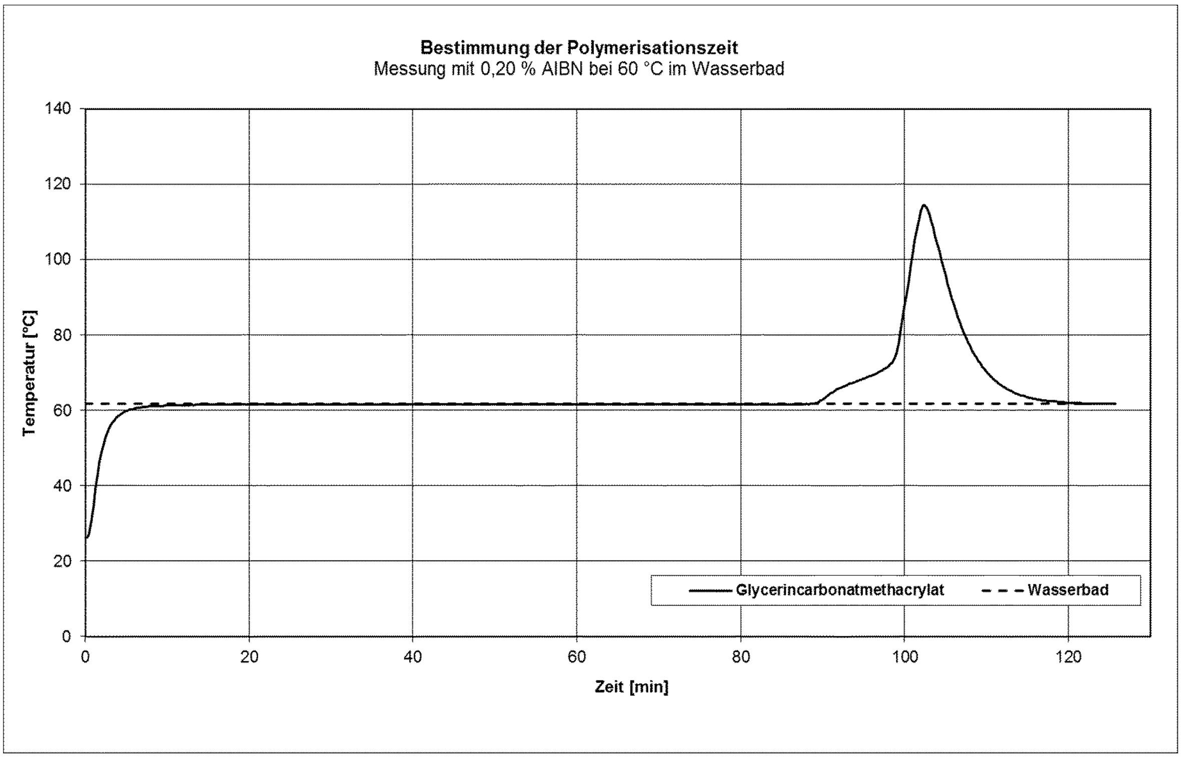
Bestimmung der Polymerisationszeit
Messung mit 0,20 % AIBN bei 60 °C im Wasserbad
Temperatur [°C]
Zeit [min]
140
120
100
80
60
40
20
0
0
20
40
60
80
100
120
Glycerincarbonatmethacrylat
Wasserbad

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 2004018389 B
- JP 2004059435 B
- DE 102011109139
- GB 690709 A
- US 4975519 A
- JP 2014044415 B


### In der Beschreibung aufgeführte Nicht-Patentliteratur


- **GUZMAN, J. et al.** *Macromolecular Symposia,* 2003, vol. 191, 177-190
- **GUZMAN.** *J. J. Polymer Sci. Part B,* 2002, vol. 40 (11), 1154-1162
- **H. MINATO.** *Bull. Chem. Soc. Jpn.,* 1969, vol. 42 (4), 1146
- **YAWS.** Handbook of Antoine Coeff. For Vapour Pressure, Electronic
- **GOTTFRIED W. RIEDEL ; GABRIELA ; TRAWIEL ; PIA.** Thermal Analysis of Plastics-Theory and Practice. Hanser Publishers, 2004